# EUROPEAN PATENT APPLICATION

(11) **EP 3 378 385 A1**
(43) Date of publication of application: **26.09.2018**
(21) Application number: 18162831.4
(22) Date of filing: 20.03.2018
(51) Int. Cl.: A61B 5/022, A61B 5/1455, A61B 5/00

(54) **SENSOR**

(30) Priority: 22.03.2017 JP 2017055651
(71) Applicant: Nihon Kohden Corporation, Shinjuku-ku Tokyo (JP)
(72) Inventor: WATANABE, Nobuyoshi, Tokorozawa-shi, Saitama (JP); ABE, Takahiro, Tokorozawa-shi, Saitama (JP); FUJISAKI, Hideki, Tokorozawa-shi, Saitama (JP); SUGIYAMA, Kumi, Tokorozawa-shi, Saitama (JP)
(74) Representative: Grünecker Patent- und Rechtsanwälte PartG mbB

(57) **Abstract**

A sensor includes a probe that acquires a blood light absorber concentration in a subject and a cuff that acquires a non-invasive blood pressure of the subject. In the sensor, the probe is configured to be attached to an interdigital area between first and second digits of a hand or foot of the subject, or a palm of the hand, and the cuff is configured to be attached to one of the first digit, the second digit, a third digit of the hand or the foot, a wrist of the hand, and an ankle of the foot.

## Description

### BACKGROUND

The present invention relates to a sensor including a probe for acquiring the blood light absorber concentration in a subject and a cuff for acquiring the non-invasive blood pressure of the subject.

JP-A-2007-029702 discloses a probe which is to be attached to the fingertip of the subject. The probe includes a light emitter and a light detector. The light detector has a light-detecting surface for detecting a light beam that is emitted from the light emitter, and that is transmitted through the fingertip of the subject. The light detector is configured so as to output a signal corresponding to the intensity of the light beam which is detected by the light-detecting surface. The wavelength of the light beam which is emitted from the light emitter is set to be absorbable by a material in blood. The volume of blood in the fingertip is changed in accordance with the pulsation, and therefore also the intensity of the light beam which is received by the light-detecting surface is changed. The signal which is output from the light detector is used for calculating vital sign such as the pulse and the arterial oxygen saturation. The arterial oxygen saturation is used as an index indicating the rate of amount of oxygen in blood as an example of the blood light absorber concentration.

In the case where measurements of the blood light absorber concentration and the non-invasive blood pressure are to be simultaneously performed on a subject, a cuff for acquiring the non-invasive blood pressure is usually wrapped around the upper arm of the subject.

In this case, a cable for a signal from the probe is drawn out from the fingertip portion of the subject, and a tube for supplying the air to the cuff is drawn out from the upper arm portion of the subject. The situation where the cable and the tube are drawn out from the separate body places of the subject may provide both the subject and the medical person with botheration.

It is an object of the invention to reduce botheration which is applied to both the subject and the medical person in the case where measurements of the blood light absorber concentration and the non-invasive blood pressure are simultaneously performed on a subject.

### SUMMARY

According to an aspect of the invention, a sensor includes:
a probe that acquires a blood light absorber concentration in a subject; and
a cuff that acquires a non-invasive blood pressure of the subject,
wherein the probe is configured to be attached to an interdigital area between first and second digits of a hand or foot of the subject, or a palm of the hand, and
the cuff is configured to be attached to one of the first digit, the second digit, a third digit of the hand or the foot, a wrist of the hand, and an ankle of the foot.

According to the configuration, both the probe for acquiring the blood light absorber concentration in the subject, and the cuff for acquiring the non-invasive blood pressure of the subject are attached to the hand or foot of the subject. Therefore, both a cable which is connected to the probe, and a tube which is connected to the cuff can be drawn out from the hand or foot of the subject. It is possible to avoid a situation where the cable and the tube are drawn out from separate body places of the subject. In the case where measurements of the blood light absorber concentration and the non-invasive blood pressure are simultaneously performed on the subject, consequently, botheration which is applied to both the subject and the medical person can be reduced.

Moreover, the probe for acquiring the blood light absorber concentration is attached to a place other than a fingertip, and hence the subject can use the fingertip even during measurement. Therefore, botheration which is provided to the subject by measurement can be further suppressed.

The acquisition of the blood light absorber concentration in the subject by the probe is performed based on a volume change of blood that is caused in accordance with pulsation of the subject in a portion to which the probe is attached. In the case where attachment positions are selected so that the probe and the cuff are placed respectively above different peripheral blood vessels, even when, in order to acquire the non-invasive blood pressure, the cuff compresses the peripheral blood vessel in the attachment portion, the pulsation of the artery in the attachment portion which is necessary for the probe to acquire the blood light absorber concentration is not inhibited. In the case where measurements of the blood light absorber concentration and the non-invasive blood pressure are simultaneously performed on the subject, therefore, the above-described botheration can be suppressed, and moreover the blood light absorber concentration can be continuously measured.

### BRIEF DESCRIPTION OF DRAWINGS

Fig. 1 illustrates an attachment state of a sensor of a first embodiment to a subject.
Fig. 2 illustrates the configuration of a probe of the sensor of Fig. 1.
Fig. 3 illustrates the configuration of a cuff of the sensor of Fig. 1.
Figs. 4A and 4B illustrate the configuration of a sensor of a second embodiment.
Figs. 5A to 5E illustrate a method of attaching the sensor of Fig. 4 to the subject.
Fig. 6 illustrates the configuration of a sensor of a first modification of the second embodiment.
Fig. 7 illustrates the configuration of a sensor of a second modification of the second embodiment.
Fig. 8 illustrates connection between the sensor of Fig. 7 and a signal processor.
Fig. 9 illustrates the configuration of a sensor of a third embodiment.
Figs. 10A to 10F illustrate a method of attaching the sensor of Fig. 9 to the subject.

### DETAILED DESCRIPTION OF EMBODIMENTS

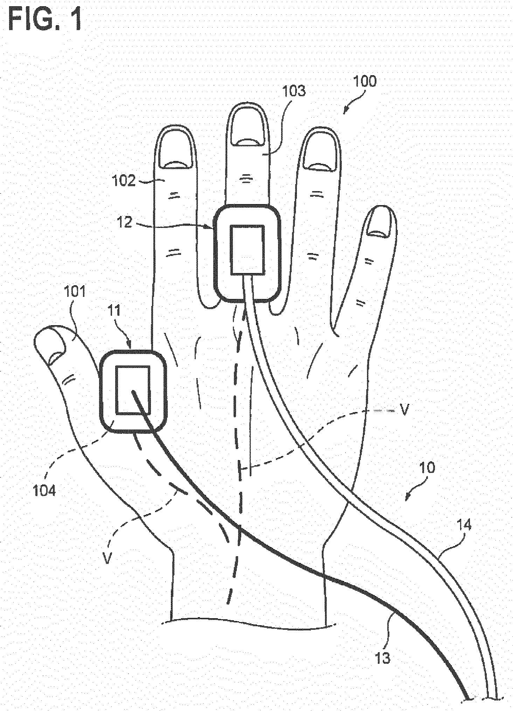

Hereinafter, embodiment examples will be described in detail with reference to the accompanying drawings. Fig. 1 illustrates a state in which a sensor 10 of a first embodiment is attached to the hand 100 of a subject.

The sensor 10 can include a probe 11 and a cuff 12. The probe 11 is a device for acquiring the arterial oxygen saturation (an example of the blood light absorber concentration) of the subject. The cuff 12 is a device for acquiring the non-invasive blood pressure of the subject.

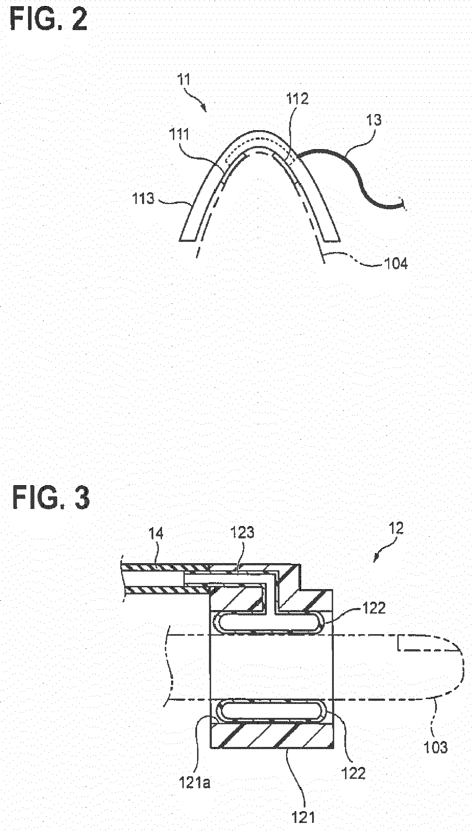

Fig. 2 schematically illustrates the configuration of the probe 11. The probe 11 can include a light emitter 111, a light detector 112, and a support member 113.

The light emitter 111 is configured so as to emit a red light beam and an infrared light beam. For example, the light emitter 111 is a semiconductor light emitting device which can emit light beams of the predetermined wavelengths. Examples of the semiconductor light emitting device are a light emitting diode (LED), a laser diode, and an organic EL device.

The light detector 112 has a light-detecting surface which detects a light beam that is transmitted through a living tissue of the subject. The light detector 112 is configured so as to output an intensity signal according to the intensity of the light beam which is received by the light-detecting surface. The volume of blood in the living tissue to which the probe 11 is attached is changed in accordance with the pulsation of the subject. Therefore, the intensity of the light beam which is detected by the light-detecting surface is changed, and also the intensity signal which is output from the light detector 112 is changed.

For example, the light detector 112 is an optical sensor having a sensitivity to the above-described predetermined wavelengths. Examples of the optical sensor are a photodiode, a phototransistor, and a photoresistor.

The light emitter 111 and the light detector 112 are supported by the support member 113. The probe 11 is configured so as to be used while being attached to an interdigital area. In the embodiment, as shown in Fig. 1, the probe 11 is attached to the interdigital area 104 between the thumb 101 (an example of the first digit) of the subject and the index finger 102 (an example of the second digit).

The sensor 10 further includes a cable 13. One end of the cable 13 is connected to the probe 11. The other end of the cable 13 is to be connected to a signal processor which is not shown. The cable 13 may include: a power supply line for supplying an electric power to the light emitter 111 and the light detector 112; a signal line for transmitting the intensity signal output from the light detector 112; and the like. The cable 13 may be inseparably integrated with the probe 11, or attachable to and detachable from the probe 11.

In the embodiment, the probe 11 is attached to the interdigital area 104 between the thumb 101 and the index finger 102. However, the probe 11 may be attached to an interdigital area between any pair of fingers in the hand 100.

Fig. 3 is a sectional view schematically illustrating the configuration of the cuff 12. The cuff 12 includes a case 121, an annular bag member 122, and an air passage 123. The case 121 has a through hole 121a. The bag member 122 is accommodated in the through hole 121a. The outer circumferential surface of the bag member 122 is fixed to the inner circumferential surface of the through hole 121a. The air passage 123 communicates with the interior of the bag member 122.

When the cuff 12 is used, a hand finger (in the example, the middle finger 103) of the subject is inserted into the through hole 121a. At this time, the inner circumferential surface of the bag member 122 surrounds the hand finger.

As shown in Fig. 1, the sensor 10 further includes a tube 14. As shown in Fig. 3, one end of the tube 14 is connected to the air passage 123 of the cuff 12. The other end of the tube 14 is to be connected to the signal processor which is not shown. The tube 14 may be inseparably integrated with the cuff 12, or attachable to and detachable from the cuff 12.

The tube 14 is used for supplying the air to the cuff 12. Specifically, the amount of the air which is supplied to the interior of the bag member 122 through the air passage 123 is adjusted based on a blood pressure measurement operation in the signal processor which is not shown. This causes the force with which the hand finger is compressed by the bag member 122 in order to acquire the non-invasive blood pressure of the subject, to be adjusted.

The cuff 12 is attached to a hand finger of the subject. In other words, the shape and dimensions of the cuff 12 which has been described with reference to Fig. 3 are configured so that the cuff is attached to a hand finger. In the embodiment, the cuff 12 is attached to the middle finger 103 (an example of the third digit) of the subject. However, the cuff 12 may be attached to any finger of the hand 100 of the subject.

In the embodiment, both the probe 11 for acquiring the arterial oxygen saturation of the subject, and the cuff 12 for acquiring the non-invasive blood pressure of the subject are attached to the hand 100 of the subject. This allows both the cable 13 which is connected to the probe 11, and the tube 14 which is connected to the cuff 12, to be drawn out from the hand 100 of the subject as shown in Fig. 1. It is possible to avoid a situation where the cable and the tube are drawn out from separate body places of the subject. In the case where measurements of the arterial oxygen saturation and the non-invasive blood pressure are simultaneously performed on the subject, therefore, botheration which is applied to both the subject and the medical person can be suppressed.

The acquisition of the arterial oxygen saturation of the subject by the probe 11 is performed based on a volume change of blood that is caused in accordance with pulsation of the subject in the interdigital area 104 to which the probe 11 is attached. In the embodiment, the probe 11 and the cuff 12 are placed respectively above different peripheral blood vessels V. Even when, in order to acquire the non-invasive blood pressure, the bag member 122 of the cuff 12 compresses the peripheral blood vessel in the middle finger 103, therefore, the pulsation of the artery in the interdigital area 104 which is necessary for the probe 11 to acquire the arterial oxygen saturation is not inhibited. In the case where measurements of the arterial oxygen saturation and the non-invasive blood pressure are simultaneously performed on the subject, consequently, the above-described botheration can be suppressed, and moreover the arterial oxygen saturation can be continuously measured.

Moreover, the probe 11 for acquiring the arterial oxygen saturation is attached to the interdigital area 104, and hence the subject can use the fingertip even during measurement. Therefore, botheration which is provided to the subject by measurement can be further suppressed.

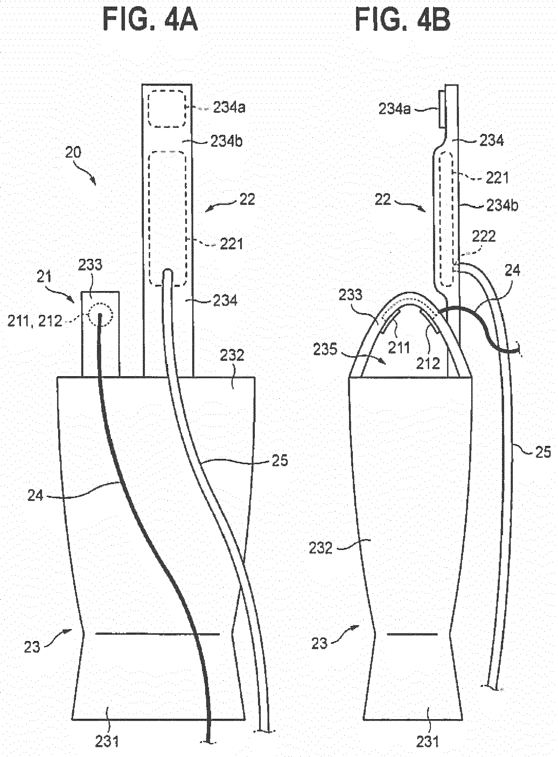

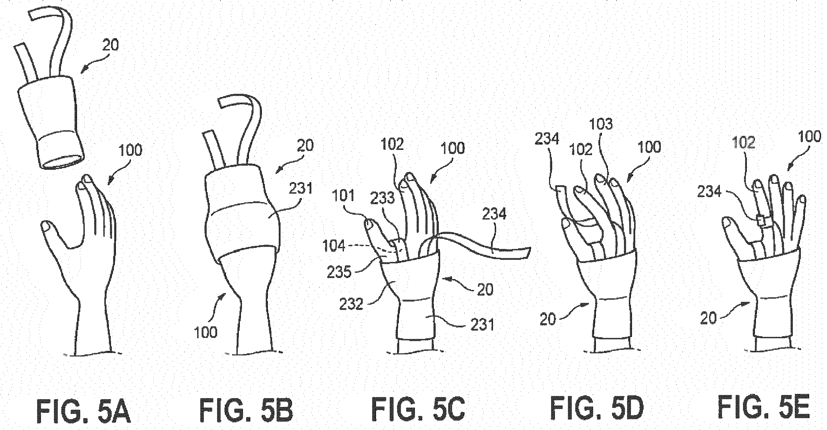

Next, a sensor 20 of a second embodiment will be described. Fig. 4A is a front view of the sensor 20, and Fig. 4B is a side view of the sensor 2. As shown in Fig. 5A to 5E, the sensor 20 is configured so as to be attached to the hand 100 of the subject.

The sensor 20 includes a probe 21, a cuff 22, and a support member 23. The probe 21 is a device for acquiring the arterial oxygen saturation of the subject. The cuff 22 is a device for acquiring the non-invasive blood pressure of the subject. The support member 23 supports the probe 21 and the cuff 22.

Specifically, the support member 23 has a first support portion 231, a second support portion 232, a third support portion 233, and a fourth support portion 234.

The first support portion 231 has a hollow tubular shape. The lower part of the first support portion 231 is opened. The shape and dimensions of the first support portion 231 are determined so that, when the sensor 20 is attached to the hand 100 of the subject, the portion is placed on the wrist.

The second support portion 232 has a hollow tubular shape. The lower part of the second support portion 232 communicates with the upper part of the first support portion 231. The upper part of the second support portion 232 is opened. The shape and dimensions of the second support portion 232 are determined so that, when the sensor 20 is attached to the hand 100 of the subject, the portion is placed on a part of the back and palm of the hand.

The third support portion 233 has a relatively short band-like appearance. Both end parts of the third support portion 233 are connected to the opening of the second support portion 232 to define a hole 235. The third support portion 233 supports the probe 21.

The fourth support portion 234 has a relatively long band-like appearance. One end part of the fourth support portion 234 is connected to one end part of the second support portion 232. The fourth support portion 234 supports the cuff 22.

The probe 21 includes a light emitter 211 and a light detector 212. The light emitter 211 is same or substantially identical in configuration with the light emitter 111 in the first embodiment, and therefore repeated description is omitted. The light detector 212 is same or substantially identical in configuration with the light detector 112 in the first embodiment, and therefore repeated description is omitted.

The sensor 20 further includes a cable 24. One end of the cable 24 is connected to the probe 21. The other end of the cable 24 is to be connected to a signal processor which is not shown. The cable 24 may include: a power supply line for supplying an electric power to the light emitter 211 and the light detector 212; a signal line for transmitting an intensity signal output from the light detector 212; and the like. The cable 24 may be inseparably integrated with the probe 21, or attachable to and detachable from the probe 21.

The cuff 22 includes a bag member 221 and an air passage 222. The bag member 221 may be formed by an independent hollow member, or by the fourth support portion 234 which is configured so as to have a bag-like shape. In the former case, the hollow member may be supported by the outer surface of the fourth support portion 234, or incorporated in the fourth support portion 234 which is configured so as to have a bag-like shape.

The sensor 20 further includes a tube 25. One end of the tube 25 is connected to the air passage 222 of the cuff 22. The other end of the tube 25 is to be connected to the signal processor which is not shown. The tube 25 may be inseparably integrated with the cuff 22, or attachable to and detachable from the cuff 22.

The tube 25 is used for supplying the air to the cuff 22. Specifically, the amount of the air which is supplied to the interior of the bag member 221 through the air passage 222 is adjusted based on a blood pressure measurement operation in the signal processor which is not shown. This causes the force with which a part of the hand 100 is compressed by the bag member 221 in order to acquire the non-invasive blood pressure of the subject, to be adjusted.

Next, a method of attaching the thus configured sensor 20 to the hand 100 of the subject will be described.

As shown in Fig. 5A and 5B, firstly, the subject inserts the hand 100 into the opening which is formed in the first support portion 231 of the support member 23.

As shown in Fig. 5C, then, the subject passes the thumb 101 through the hole 235. The other fingers are passed through the opening of the second support portion 232. The first support portion 231 is placed on the wrist. The second support portion 232 is placed on a part of the back and palm of the hand. The third support portion 233 is placed on the interdigital area 104 between the thumb 101 (an example of the first digit) and the index finger 102 (an example of the second digit).

As shown in Fig. 5D, then, the subject passes the fourth support portion 234 between the index finger 102 and the middle finger 103. As shown in Fig. 5E, thereafter, the fourth support portion 234 is wound around the index finger 102, thereby completing the attachment of the sensor 20 to the hand 100.

As shown in Fig. 4B, the fourth support portion 234 has a hook surface 234a and loop surface 234b which form a hook and loop fastener. The hook surface 234a is fixed at an adequate position of the loop surface 234b to secure the cuff 22 to the index finger 102.

The cuff 22 can be attached to a finger of the hand 100 of the subject. In this case, the position of the fourth support portion 234 can be appropriately determined.

In the embodiment, both the probe 21 for acquiring the arterial oxygen saturation of the subject, and the cuff 22 for acquiring the non-invasive blood pressure of the subject are attached to the hand 100 of the subject. Therefore, both the cable 24 which is connected to the probe 21, and the tube 25 which is connected to the cuff 22 can be drawn out from the hand 100 of the subject. It is possible to avoid a situation where the cable and the tube are drawn out from separate body places of the subject. In the case where measurements of the arterial oxygen saturation and the non-invasive blood pressure are simultaneously performed on the subject, therefore, botheration which is applied to both the subject and the medical person can be suppressed.

The acquisition of the arterial oxygen saturation of the subject by the probe 21 is performed based on a volume change of blood that is caused in accordance with pulsation of the subject in the interdigital area 104 to which the probe 21 is attached. In the embodiment, the probe 21 and the cuff 22 are placed respectively above different peripheral blood vessels. Even when, in order to acquire the non-invasive blood pressure, the bag member 221 of the cuff 22 compresses the peripheral blood vessel in the index finger 102, therefore, the pulsation of the artery in the interdigital area 104 which is necessary for the probe 21 to acquire the arterial oxygen saturation is not inhibited. In the case where measurements of the arterial oxygen saturation and the non-invasive blood pressure are simultaneously performed on the subject, therefore, the above-described botheration can be suppressed, and moreover the arterial oxygen saturation can be continuously measured.

Moreover, the probe 21 for acquiring the arterial oxygen saturation is attached to the interdigital area 104, and hence the subject can use the fingertip even during measurement. Therefore, botheration which is provided to the subject by measurement can be further suppressed.

In the embodiment, the probe 21 is supported by the third support portion 233 which is a part of the support member 23, and the cuff 22 is supported by the fourth support portion 234 which is similarly a part of the support member 23. Namely, the common support member 23 supports both the probe 21 and the cuff 22.

According to the configuration, the efficiency of the work of attaching the probe 21 and the cuff 22 can be enhanced as compared with a configuration where a probe and a cuff are supported respectively by individual support members. Moreover, also the cable 24 which is connected to the probe 21, and the tube 25 which is connected to the cuff 22 are supported by the common support member 23. Therefore, the drawn-out directions of the cable 24 and tube 25 which are drawn out from different places of the hand 100 can be easily aligned with each other. In the case where measurements of the arterial oxygen saturation and the non-invasive blood pressure are simultaneously performed on the subject, therefore, botheration which is applied to both the subject and the medical person can be further suppressed.

In the embodiment, the support member 23 is made of a stretchable material.

According to the configuration, the probe 21 and the cuff 22 can be closely attached to respective attachment portions. Therefore, the accuracy of the arterial oxygen saturation and non-invasive blood pressure which are acquired can be improved.

In the embodiment, the second support portion 232 and third support portion 233 of the support member 23 define the hole 235. When the support member 23 is attached to the hand 100 of the subject, the thumb 101 of the subject is passed through the hole 235. The light emitter 211 and light detector 212 of the probe 21 are supported by the third support portion 233 which is adjacent to the hole 235.

According to the configuration, the probe 21 is enabled to be located on the interdigital area 104 between the thumb 101 and index finger 102 which are adjacent to the hole 235, simply by causing the thumb 101 to pass through the hole 235. Therefore, the efficiency of the work of attaching the probe 21 can be enhanced. In the case where measurements of the arterial oxygen saturation and the non-invasive blood pressure are simultaneously performed on the subject, consequently, botheration which is applied to both the subject and the medical person can be further suppressed.

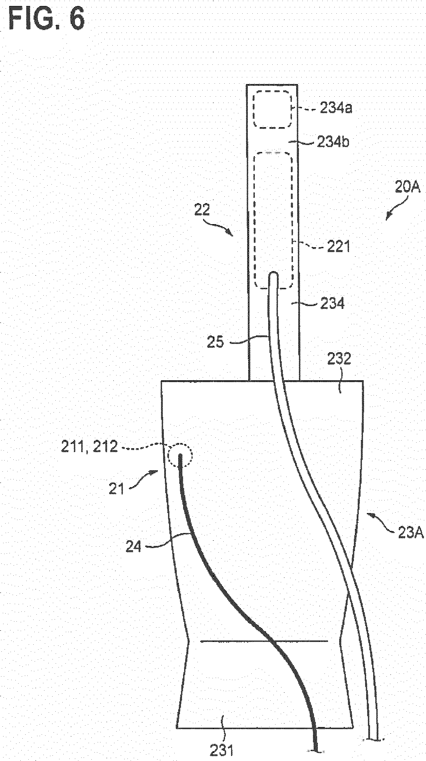

Fig. 6 illustrates a sensor 20A of a first modification of the second embodiment. Components which are substantially identical with those of the sensor 20 are denoted by the same reference numerals, and repeated description is omitted.

The sensor 20A includes a support member 23A. The support member 23A includes the first support portion 231, the second support portion 232, and the fourth support portion 234. Namely, the support member 23A does not include a configuration corresponding to the third support portion 233 of the support member 23.

In the modification, the probe 21 is supported by the second support portion 232. More specifically, the light emitter 211 and light detector 212 of the probe 21 are supported by the inner surface of the second support portion 232 having a hollow tubular shape.

When the sensor 20A is attached to the hand 100 of the subject, the probe 21 is placed on a palm portion of the hand 100. More specifically, the probe 21 is placed on a palm portion which is positioned between the thumb and the wrist, or that which is positioned between the little finger and the wrist.

Also according to the configuration, both the probe 21 for acquiring the arterial oxygen saturation of the subject, and the cuff 22 for acquiring the non-invasive blood pressure of the subject are attached to the hand 100 of the subject. Therefore, both the cable 24 which is connected to the probe 21, and the tube 25 which is connected to the cuff 22 can be drawn out from the hand 100 of the subject. It is possible to avoid a situation where the cable and the tube are drawn out from separate body places of the subject. In the case where measurements of the arterial oxygen saturation and the non-invasive blood pressure are simultaneously performed on the subject, therefore, botheration which is applied to both the subject and the medical person can be suppressed.

The acquisition of the arterial oxygen saturation of the subject by the probe 21 is performed based on a volume change of blood that is caused in accordance with pulsation of the subject in the palm portion to which the probe 21 is attached. In the embodiment, the probe 21 and the cuff 22 are placed respectively above different peripheral blood vessels. Even when, in order to acquire the non-invasive blood pressure, the bag member 221 of the cuff 22 compresses the peripheral blood vessel in the finger, the pulsation of the artery in the palm portion which is necessary for the probe 21 to acquire the arterial oxygen saturation is not inhibited. In the case where measurements of the arterial oxygen saturation and the non-invasive blood pressure are simultaneously performed on the subject, therefore, the above-described botheration can be suppressed, and moreover the arterial oxygen saturation can be continuously measured.

Moreover, the probe 21 for acquiring the arterial oxygen saturation is attached to the palm portion, and hence the subject can use the fingertip even during measurement. Therefore, botheration which is provided to the subject by measurement can be further suppressed.

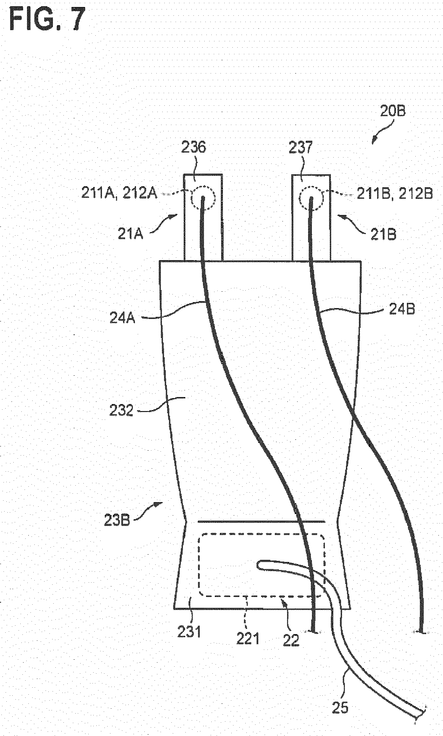

Fig. 7 illustrates a sensor 20B of a second modification of the second embodiment. Components which are substantially identical with those of the sensor 20 are denoted by the same reference numerals, and repeated description is omitted.

The sensor 20B includes a first probe 21A, a second probe 21B, the cuff 22, and a support member 23B.

The first probe 21A includes a first light emitter 211A and a first light detector 212A. The configuration of the first light emitter 211A is substantially identical with that of the light emitter 111 in the first embodiment, and therefore repeated description is omitted. The configuration of the first light detector 212A is substantially identical with that of the light detector 112 in the first embodiment, and therefore repeated description is omitted.

The second probe 21B includes a second light emitter 211B and a second light detector 212B. The configuration of the second light emitter 211B is same or substantially identical with that of the light emitter 111 in the first embodiment, and therefore repeated description is omitted. The configuration of the second light detector 212B is substantially identical with that of the light detector 112 in the first embodiment, and therefore repeated description is omitted.

The support member 23B includes the first support portion 231, the second support portion 232, a first probe support portion 236, and a second probe support portion 237. The first probe support portion 236 supports the first light emitter 211A and the first light detector 212A. The second probe support portion 237 supports the second light emitter 211B and the second light detector 212B. The configurations of the first probe support portion 236 and the second probe support portion 237 are identical with the configuration of the third support portion 233 shown in Fig. 4B, and therefore repeated description is omitted.

The sensor 20B further includes a first cable 24A. One end of the first cable 24A is connected to the first probe 21A. The first cable 24A may include: a power supply line for supplying an electric power to the first light emitter 211A and the first light detector 212A; a signal line for transmitting an intensity signal output from the first light detector 212A; and the like. The first cable 24A may be inseparably integrated with the first probe 21A, or attachable to and detachable from the first probe 21A.

The sensor 20B further includes a second cable 24B. One end of the second cable 24B is connected to the second probe 21B. The second cable 24B may include: a power supply line for supplying an electric power to the second light emitter 211B and the second light detector 212B; a signal line for transmitting an intensity signal output from the second light detector 212B; and the like. The second cable 24B may be inseparably integrated with the second probe 21B, or attachable to and detachable from the second probe 21B.

The cuff 22 is supported by the first support portion 231 of the support member 23B.

When the sensor 20B is attached to the hand 100 of the subject, the first probe 21A is placed on an interdigital area between two fingers (for example, the thumb and the index finger) of the subject which constitute a first pair. On the other hand, the second probe 21B is placed on an interdigital area between two fingers (for example, the middle finger and the medical finger) of the subject which constitute a second pair. The cuff 22 is placed on the wrist of the subject.

Also according to the configuration, all the first probe 21A and second probe 21B for acquiring the arterial oxygen saturation of the subject, and the cuff 22 for acquiring the non-invasive blood pressure of the subject are attached to the hand 100 of the subject. Therefore, all the first cable 24A which is connected to the first probe 21A, the second cable 24B which is connected to the second probe 21B, and the tube 25 which is connected to the cuff 22 are drawn out from the hand 100 of the subject. It is possible to avoid a situation where the cables and the tube are drawn out from separate body places of the subject. In the case where measurements of the arterial oxygen saturation and the non-invasive blood pressure are simultaneously performed on the subject, therefore, botheration which is applied to both the subject and the medical person can be suppressed.

Moreover, the first probe 21A and second probe 21B for acquiring the arterial oxygen saturation are attached to the interdigital areas, and hence the subject can use the fingertip even during measurement. Therefore, botheration which is provided to the subject by measurement can be further suppressed.

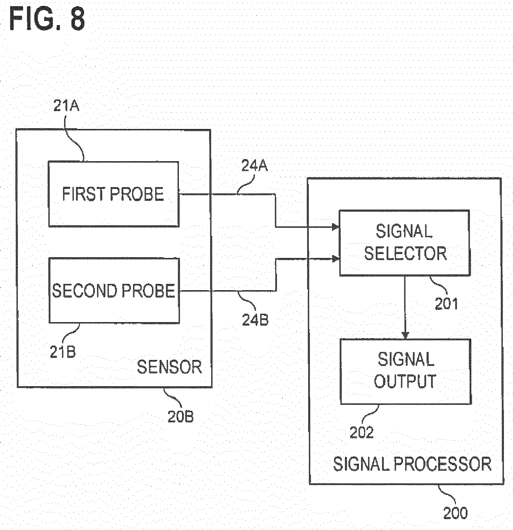

As shown in Fig. 8, both the first cable 24A which is connected to the first probe 21A, and the second cable 24B which is connected to the second probe 21B are connected to a signal processor 200.

The signal processor 200 includes a signal selector 201 and a signal output 202. The signal output from the first light detector 212A of the first probe 21A is input to the signal selector 201 through the first cable 24A. The signal output from the second light detector 212B of the second probe 21B is input to the signal selector 201 through the second cable 24B.

The signal selector 201 is configured so as to select a higher quality signal from the signal supplied from the first probe 21A, and that supplied from the second probe 21B, and output the selected signal to the signal output 202. The quality level of a signal is defined by, for example, the level of the S/N ratio or the like.

According to the configuration, a process such as that signals for calculating the arterial oxygen saturation of the subject are acquired through a plurality of interdigital areas, and the optimum signal is selected from the signals can be performed.

The placement of the cuff 22 in the example can be applied also to the sensor 20 and sensor 20A which have been described above.

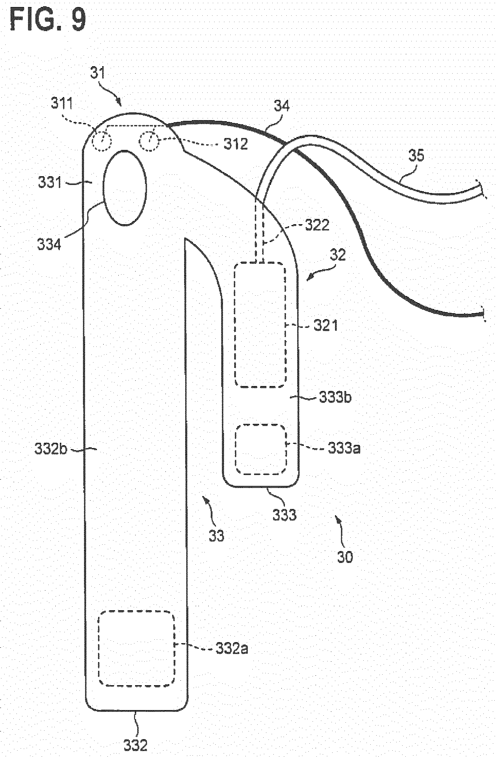

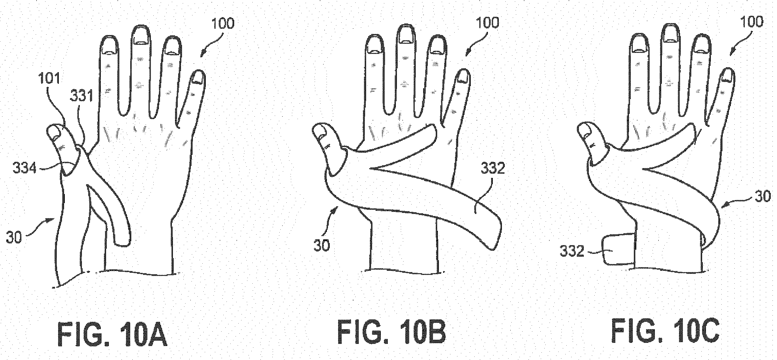

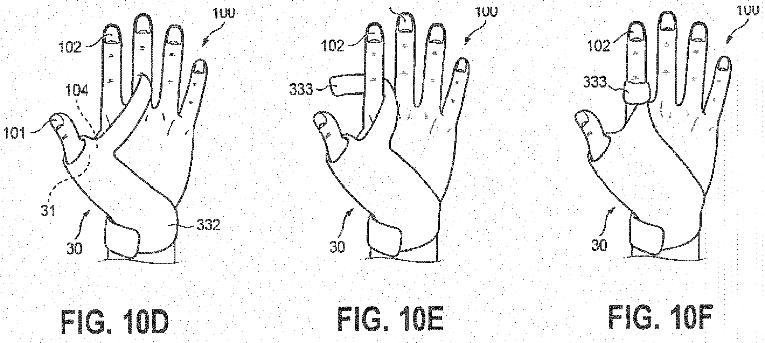

Fig. 9 illustrates a sensor 30 of a third embodiment. As shown in Fig. 10A to 10F, the sensor 30 is configured so as to be attached to the hand 100 of the subject.

The sensor 30 includes a probe 31, a cuff 32, and a support member 33. The probe 31 is a device for acquiring the arterial oxygen saturation of the subject. The cuff 32 is a device for acquiring the non-invasive blood pressure of the subject. The support member 33 supports the probe 31 and the cuff 32.

Specifically, the support member 33 has a basal end portion 331, a first band portion 332, and a second band portion 333.

The basal end portion 331 has a hole 334. The basal end portion 331 supports the probe 31 at a position adjacent to the hole 334.

The first band portion 332 and the second band portion 333 elongate in different directions from the basal end portion 331. The length dimension of the first band portion 332 is larger than that of the second band portion 333. The second band portion 333 supports the cuff 32.

The probe 31 includes a light emitter 311 and a light detector 312. The light emitter 311 is same or substantially identical in configuration with the light emitter 111 in the first embodiment, and therefore repeated description is omitted. The light detector 312 is same or substantially identical in configuration with the light detector 112 in the first embodiment, and therefore repeated description is omitted.

The sensor 30 further includes a cable 34. One end of the cable 34 is connected to the probe 31. The other end of the cable 34 is to be connected to a signal processor which is not shown. The cable 34 may include: a power supply line for supplying an electric power to the light emitter 311 and the light detector 312; a signal line for transmitting an intensity signal output from the light detector 312; and the like. The cable 34 may be inseparably integrated with the probe 31, or attachable to and detachable from the probe 31.

The cuff 32 includes a bag member 321 and an air passage 322. The bag member 321 may be formed by an independent hollow member, or by the second band portion 333 which is configured so as to have a bag-like shape. In the former case, the hollow member may be supported by the outer surface of the second band portion 333, or incorporated in the second band portion 333 which is configured so as to have a bag-like shape.

The sensor 30 further includes a tube 35. One end of the tube 35 is connected to the air passage 322 of the cuff 32. The other end of the tube 35 is to be connected to the signal processor which is not shown. The tube 35 may be inseparably integrated with the cuff 32, or attachable to and detachable from the cuff 32.

The tube 35 is used for supplying the air to the cuff 32. Specifically, the amount of the air which is supplied to the interior of the bag member 321 through the air passage 322 is adjusted based on a blood pressure measurement operation in the signal processor which is not shown. This causes the force with which a part of the hand 100 is compressed by the bag member 321 in order to acquire the non-invasive blood pressure of the subject, to be adjusted.

Next, a method of attaching the thus configured sensor 30 to the hand 100 of the subject will be described.

As shown in Fig. 10A, firstly, the subject inserts the thumb 101 (an example of the first digit) into the hole 334 which is formed in the basal end portion 331 of the support member 33.

As shown in Fig. 10B to 10D, next, the subject winds the first band portion 332 of the support member 33 around the wrist to fix the member to the wrist. As shown in Fig. 9, the first band portion 332 has a hook surface 332a and loop surface 332b which form a hook and loop fastener. The hook surface 332a is fixed at an adequate position of the loop surface 332b, whereby the probe 31 is pulled in the direction in which the first band portion 332 elongates, and the light emitter 311 and the light detector 312 are closely contacted with the interdigital area 104 between the thumb 101 and the index finger 102 (an example of the second digit).

As shown in Fig. 10E, then, the subject passes the second band portion 333 between the index finger 102 and the middle finger 103. As shown in Fig. 10F, thereafter, the second band portion 333 is wound around the index finger 102, thereby completing the attachment of the sensor 30 to the hand 100.

As shown in Fig. 9, the second band portion 333 has a hook surface 333a and loop surface 333b which form a hook and loop fastener. The hook surface 333a is fixed at an adequate position of the loop surface 333b, whereby the cuff 32 is fixed to the index finger 102.

The probe 31 can be attached to an interdigital area between arbitrary paired fingers of the hand 100 of the subject. In this case, the shapes and dimensions of the basal end portion 331, the first band portion 332, and the hole 334 can be appropriately determined.

Similarly, the cuff 32 can be attached to an arbitrary finger of the hand 100 of the subject. In this case, the shape and dimensions of the second band portion 333 can be appropriately determined.

In the embodiment, both the probe 31 for acquiring the arterial oxygen saturation of the subject, and the cuff 32 for acquiring the non-invasive blood pressure of the subject are attached to the hand 100 of the subject. This allows both the cable 34 which is connected to the probe 31, and the tube 35 which is connected to the cuff 32, to be drawn out from the hand 100 of the subject. It is possible to avoid a situation where the cable and the tube are drawn out from separate body places of the subject. In the case where measurements of the arterial oxygen saturation and the non-invasive blood pressure are simultaneously performed on the subject, therefore, botheration which is applied to both the subject and the medical person can be suppressed.

The acquisition of the arterial oxygen saturation of the subject by the probe 31 is performed based on a volume change of blood that is caused in accordance with pulsation of the subject in the interdigital area 104 to which the probe 31 is attached. In the embodiment, the probe 31 and the cuff 32 are placed respectively above different peripheral blood vessels. Even when, in order to acquire the non-invasive blood pressure, the bag member 321 of the cuff 32 compresses the peripheral blood vessel in the index finger 102, therefore, the pulsation of the artery in the interdigital area 104 which is necessary for the probe 31 to acquire the arterial oxygen saturation is not inhibited. In the case where measurements of the arterial oxygen saturation and the non-invasive blood pressure are simultaneously performed on the subject, consequently, the above-described botheration can be suppressed, and moreover the arterial oxygen saturation can be continuously measured.

Moreover, the probe 31 for acquiring the arterial oxygen saturation is attached to the interdigital area 104, and hence the subject can use the fingertip even during measurement. Therefore, botheration which is provided to the subject by measurement can be further suppressed.

In the embodiment, the probe 31 is supported by the basal end portion 331 which is a part of the support member 33, and the cuff 32 is supported by the second band portion 333 which is similarly a part of the support member 33. Namely, the common support member 33 supports both the probe 31 and the cuff 32.

According to the configuration, the efficiency of the work of attaching the probe 31 and the cuff 32 can be enhanced as compared with a configuration where a probe and a cuff are supported respectively by individual support members. Moreover, also the cable 34 which is connected to the probe 31, and the tube 35 which is connected to the cuff 32 are supported by the common support member 33. Therefore, the drawn-out directions of the cable 34 and tube 35 which are drawn out from different places of the hand 100 can be easily aligned with each other. In the case where measurements of the arterial oxygen saturation and the non-invasive blood pressure are simultaneously performed on the subject, therefore, botheration which is applied to both the subject and the medical person can be further suppressed.

The support member 33 can be formed by a stretchable material.

According to the configuration, the probe 31 and the cuff 32 can be closely attached to respective attachment portions. Therefore, the accuracy of the arterial oxygen saturation and non-invasive blood pressure which are acquired can be improved.

In the embodiment, the hole 334 is formed in the basal end portion 331 of the support member 33. When the support member 23 is attached to the hand 100 of the subject, the thumb 101 of the subject is passed through the hole 334. The light emitter 311 and light detector 312 of the probe 31 are supported at positions which are adjacent to the hole 334 in the basal end portion 331.

According to the configuration, the probe 31 is enabled to be located on the interdigital area 104 between the thumb 101 and index finger 102 which are adjacent to the hole 334, simply by causing the thumb 101 to pass through the hole 334. Therefore, the efficiency of the work of attaching the probe 31 can be enhanced. In the case where measurements of the arterial oxygen saturation and the non-invasive blood pressure are simultaneously performed on the subject, consequently, botheration which is applied to both the subject and the medical person can be further suppressed.

The above-described embodiments facilitate understanding of the invention, and do not limit the invention. It is obvious that the invention may be changed or improved without departing from the spirit of the invention, and its equivalents are included within the scope of the invention.

In the above-described embodiments, the probe for acquiring the arterial oxygen saturation is attached to an interdigital area of the hand of the subject. However, the probe may be attached to an interdigital area of the foot of the subject. In the above-described embodiments, in order to acquire the non-invasive blood pressure, the cuff is attached to a finger of the hand or wrist of the subject. However, the cuff may be attached to a toe of the foot or ankle of the subject.

In the above-described embodiments, the probe is used for acquiring the arterial oxygen saturation. However, the probe may have a configuration for acquiring the concentration of carboxyhemoglobin, methemoglobin, or the like which is a blood light absorber.

In the above-described embodiments, the light emitter 111 is configured so as to emit a red light beam and an emission light beam. However, the light emitter 111 may be configured so as to emit a blue light beam, a green light beam, an orange light beam, a red-orange light beam, or the like.

## Claims

1. A sensor comprising:
a probe that acquires a blood light absorber concentration in a subject; and
a cuff that acquires a non-invasive blood pressure of the subject,
wherein the probe is configured to be attached to an interdigital area between first and second digits of a hand or foot of the subject, or a palm of the hand, and
the cuff is configured to be attached to one of the first digit, the second digit, a third digit of the hand or the foot, a wrist of the hand, and an ankle of the foot.

2. The sensor according to claim 1 further comprising a support member which is common to and supports the probe and the cuff.

3. The sensor according to claim 2, wherein the support member is made of a stretchable material.

4. The sensor according to claims 1 or 3, wherein the sensor includes a support member that has a hole through which the first digit or the second digit is passable, and
the probe is supported at a position adjacent to the hole.

5. The sensor according to claim 2, wherein the support member includes a hole through which the first digit or the second digit is passable, and
the probe is supported at a position adjacent to the hole.

6. The sensor according to any one of claims 1 to 5, wherein the probe includes:
a first probe that is configured to be attached to a first interdigital area between two digits of the subject; and
a second probe which is configured to be attached to a second interdigital area between two digits of the subject.
